# EUROPEAN PATENT SPECIFICATION

(11) **EP 3 337 817 B1**
(45) Date of publication and mention of the grant of the patent: **15.09.2021**
(21) Application number: 16753879.2
(22) Date of filing: 11.08.2016
(51) Int. Cl.: C07K 16/06

(54) **METHOD FOR THE REDUCTION OF HOST CELL PROTEINS IN AFFINITY CHROMATOGRAPHY**
VERFAHREN ZUR REDUKTION VON WIRTSZELLENPROTEINEN IN DER AFFINITÄTSCHROMATOGRAFIE
PROCÉDÉ POUR LA RÉDUCTION DE PROTÉINES DE CELLULE HÔTE DANS LA CHROMATOGRAPHIE D'AFFINITÉ

(30) Priority: 21.08.2015 EP 15181903
(43) Date of publication of application: 27.06.2018
(73) Proprietor: F. Hoffmann-La Roche AG, 4070 Basel (CH)
(72) Inventor: FALKENSTEIN, Roberto, 80689 Munich (DE); KOEHNLEIN, Wolfgang, 83671 Benediktbeuern (DE); SCHWENDNER, Klaus, 82362 Weilheim (DE); SPENSBERGER, Bernhard, 82390 Eberfing (DE)
(74) Representative: Burger, Alexander
(86) International application number: PCT/EP2016/069163
(87) International publication number: WO 2017/032611

(56) References cited:
- EP-A1- 2 583 973
- WO-A1-2012/164046
- WO-A1-2015/038888

## Description

The present invention generally relates to the field of purification of polypeptides. The present invention in particular relates to the reduction of host cell proteins like phospholipase B-like 2 (PLBL2) or Clusterin in solutions containing antibodies.

### Background of the Invention

Proteins and especially immunoglobulins play an important role in today's medical portfolio. For human application every therapeutic protein has to meet distinct criteria. To ensure the safety of biopharmaceutical agents to humans by-products accumulating during the production process have to be removed especially. To fulfill the regulatory specifications one or more purification steps have to follow the manufacturing process. Among other things, purity, throughput, and yield play an important role in determining an appropriate purification process.

Different methods are well established and widespread used for protein purification, such as affinity chromatography (e.g. protein A or protein G affinity chromatography, single chain Fv ligand affinity chromatography), ion exchange chromatography (e.g. cation exchange (sulfopropyl or carboxymethyl resins), anion exchange (amino ethyl resins) and mixed-mode ion exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis).

For the purification of recombinantly produced immunoglobulins often a combination of different column chromatography steps is employed. During the purification non-immunoglobulin contaminants such as host cell protein and host cell DNA as well as endotoxins and viruses are depleted. Therefore, generally an affinity chromatography step, like protein A affinity chromatography is followed by one or more additional separation steps. In general, high conductivity buffers are described to be employed in wash steps of affinity chromatrography methods.

In US 6,127,526 a method for purifying proteins by Protein A chromatography is described which comprises the steps of: (a) adsorbing the protein to Protein A immobilized on a solid phase comprising silica or glass; (b) removing contaminants bound to the solid phase by washing the solid phase with a hydrophobic electrolyte solvent; and (c) recovering the protein from the solid phase.

In WO2011/038894 a protein A chromatography method with a pronounced depletion of host cell protein and DNA by specific wash steps prior to the recovery of the immunoglobulin from the protein A chromatographic material is reported.

In WO2013/177118 compositions and methods for the isolation and purification of antibodies from a sample matrix are reported.

In WO2013/033517 methods for separating a polypeptide of interest (such as an antibody) from a virus are reported.

A method for purifying a protein, including one or more chromatographic processes, in which an amino acid; or a dipeptide, an oligopeptide, or a polyamino acid thereof is included in a buffer solution used in at least one chromatographic process (equilibration buffer, wash buffer, and elution buffer), thereby purifying a high-purity protein with a very small quantity of the impurity (e.g., polymers or host cell proteins) is reported in EP2583973.

In WO2015/038888 methods and compositions comprising purified recombinant polypeptides are reported.

WO2012/164046 reports a washing method for affinity chromatography in which a wash solution comprising arginine, or an arginine derivative, at pH greater than 8.0, is effective in removing impurities without the presence of a nonbuffering salt, while simultaneously increasing product concentration in the eluate and maintaining a high percent yield of recovered product.

### Summary of the Invention

Herein is reported a method for the production of an antibody with reduced content of specific CHO host cell proteins by purifying the antibody with an affinity chromatography step.

In more detail it has been found that by the method of the current invention which uses an aqueous solution that comprises Histidine in a wash step of an affinity chromatography prior to the recovery of an antibody from the chromatographic material, the content of a host cell protein in a solution comprising an antibody can be reduced. Especially the content of phospholipases (in particular phospholipase B-like 2 (PLBL2)) can be reduced.

One aspect as reported herein is the use of an aqueous solution comprising Histidine in a wash step of a protein A chromatography for reducing the content of a (specific) host cell protein wherein the protein A chromatography is used to purify a human IgG4 or IgG1 isotype antibody.

One aspect as reported herein is a method for producing a human IgG4 or IgG1 isotype antibody comprising the following steps
a) cultivating a cell comprising a nucleic acid encoding a human IgG4 or IgG1 isotype antibody,
b) recovering the human IgG4 or IgG1 isotype antibody from the cell or the cultivation medium,
c) contacting the human IgG4 or IgG1 isotype antibody with a protein A chromatography material,
d) washing the protein A chromatography material with an aqueous solution comprising Histidine,
e) recovering the human IgG4 or IgG1 isotype antibody from the protein A chromatography material
and thereby producing the human IgG4 or IgG1 isotype antibody.

One aspect as reported herein is a method for purifying a human IgG4 or IgG1 isotype antibody from a sample comprising the steps of
a) providing a sample comprising a human IgG4 or IgG1 isotype antibody,
b) purifying the human IgG4 or IgG1 isotype antibody with a protein A chromatography method/step, comprising washing the protein A chromatography material with an aqueous solution comprising Histidine.

In one embodiment of all aspects the aqueous solution comprises about 50 mM to about 400 mM Histidine. In one embodiment of all aspects the aqueous solution comprises about 200 mM Histidine.

In one embodiment of all aspects the aqueous solution comprises Histidine and Tris. In one embodiment of all aspects the aqueous solution comprises 50mM to about 400 mM Histidine and about 800 mM to about 1200 mM Tris. In one embodiment of all aspects the aqueous solution comprises about 200 mM Histidine and about 1000 mM Tris.

In one embodiment of all aspects the protein A chromatography additionally comprises a wash step with a low conductivity aqueous solution. This low conductivity aqueous solution does not comprise Histidine.

In one embodiment of all aspects the low conductivity aqueous solution has a conductivity value of about 0.5 mS/cm or less.

In one embodiment of all aspects the (specific) host cell protein is phospholipase B-like 2 (PLBL2) or Clusterin.

In one embodiment of all aspects the low conductivity aqueous solution comprises about 0.1 to about 8 mM Tris. In one embodiment of all aspects the low conductivity aqueous solution comprises about 0.05 to about 2 mM potassium phosphate.

In one embodiment of all aspects the low conductivity aqueous solution has a pH of about 7 or higher.

In one embodiment of all aspects the human IgG4 isotype antibody is an antibody against P-selectin or an antibody against factor IXa and factor X. In one embodiment of all aspects the human IgG1 isotype antibody is an antibody against amyloid beta or an antibody against Her2 or an antibody against Ang2 and VEGF-A or an antibody against carcinoembryonic antigen (CEA) and CD3.

### Detailed Description of the Invention

Herein is reported an improved affinity chromatography method and use comprising the washing of the affinity chromatography material with an aqueous solution comprising Histidine.

It has been found that host cell proteins can be reduced with a wash step with a aqueous solution comprising Histidine, when this wash step is used in an affinity chromatography step, e.g. a protein A chromatrography step. The affinity chromatography step is used in a purification or production method for antibodies. The wash step with an aqueous solution comprising Histidine is particularly effective to reduce the content of phospholipase B-like 2 (PLBL2). The effect can be reinforced if additionally a wash step with a low conductivity aqueous solution is used.

One aspect as reported herein is the use of an aqueous solution comprising Histidine in a wash step of an affinity chromatography for reducing the content of a (specific) host cell protein wherein the affinity chromatography is used to purify a human IgG isotype antibody.

One aspect as reported herein is the use of an aqueous solution comprising Histidine and Tris in a wash step of an affinity chromatography for reducing the content of a (specific) host cell protein wherein the affinity chromatography is used to purify a human IgG isotype antibody, wherein the aqueous solution has a pH of about 6.5 or higher.

One aspect as reported herein is a method for producing a human IgG isotype antibody comprising the following steps
a) cultivating a cell comprising a nucleic acid encoding a human IgG isotype antibody,
b) recovering the human IgG isotype antibody from the cell or the cultivation medium,
c) contacting the human IgG isotype antibody with a protein A chromatography material,
d) washing the protein A chromatography material with an aqueous solution comprising Histidine and Tris that has a pH of about 6.5 or higher,
e) recovering the human IgG isotype antibody from the protein A chromatography material
and thereby producing the human IgG isotype antibody.

One aspect as reported herein is a method for purifying a human IgG isotype antibody from a sample comprising the steps of
a) providing a sample comprising a human IgG isotype antibody,
b) purifying the human IgG isotype antibody with a protein A chromatography method/step, comprising washing the protein A chromatography material with an aqueous solution comprising Histidine and Tris that has a pH of about 6.5 or higher.

One aspect as reported herein is a method for producing a human IgG isotype antibody comprising the following steps
a) cultivating a cell comprising a nucleic acid encoding a human IgG isotype antibody,
b) recovering the human IgG isotype antibody from the cell or the cultivation medium,
c) contacting (a solution comprising) the human IgG isotype antibody with an affinity chromatography material,
d) washing the affinity chromatography material with an aqueous solution comprising Histidine, while at least 90% of the bispecific antibody remains bound to the affinity chromatography material,
e) recovering the human IgG isotype antibody from the affinity chromatography material,
and thereby producing the human IgG isotype antibody.

One aspect as reported herein is a method for purifying a human IgG isotype antibody from a sample comprising the steps of
a) providing a (buffered aqueous) sample comprising a human IgG isotype antibody,
b) purifying the human IgG isotype antibody with an affinity chromatography method/step, comprising washing the affinity chromatography material with an aqueous solution comprising Histidine, while at least 90% of the bispecific antibody remains bound to the affinity chromatography material.

One aspect as reported herein is a method for producing a human IgG isotype antibody comprising the following steps
a) cultivating a cell comprising a nucleic acid encoding a human IgG isotype antibody,
b) recovering the human IgG isotype antibody from the cell or the cultivation medium,
c) contacting (a solution comprising) the human IgG isotype antibody with an affinity chromatography material,
d) washing the affinity chromatography material with an aqueous solution comprising Histidine and Tris that has a pH of about 6.5 or higher, while at least 90% of the bispecific antibody remains bound to the affinity chromatography material,
e) recovering the human IgG isotype antibody from the affinity chromatography material,
and thereby producing the human IgG isotype antibody.

One aspect as reported herein is a method for purifying a human IgG isotype antibody from a sample comprising the steps of
a) providing a (buffered aqueous) sample comprising a human IgG isotype antibody,
b) purifying the human IgG isotype antibody with an affinity chromatography method/step, comprising washing the affinity chromatography material with an aqueous solution comprising Histidine and Tris that has a pH of about 6.5 or higher, while at least 90% of the bispecific antibody remains bound to the affinity chromatography material.

In one embodiment the affinity chromatography is used to purify a human IgG isotype antibody. In one preferred embodiment the affinity chromatography is used to purify a human IgG4 or IgG1 isotype antibody.

One aspect as reported herein is the use of an aqueous solution comprising Histidine in a wash step of a protein A chromatography for reducing the content of a (specific) host cell protein wherein the protein A chromatography is used to purify a human IgG4 or IgG1 isotype antibody.

One aspect as reported herein is a method for producing a human IgG4 or IgG1 isotype antibody comprising
a) cultivating a cell comprising a nucleic acid encoding a human IgG4 or IgG1 isotype antibody,
b) recovering the human IgG4 or IgG1 isotype antibody from the cell or the cultivation medium,
c) contacting the human IgG4 or IgG1 isotype antibody with a protein A chromatography material,
d) washing the protein A chromatography material with an aqueous solution comprising Histidine, while at least 90% of the bispecific antibody remains bound to the affinity chromatography material,
e) recovering the human IgG4 or IgG1 isotype antibody from the protein A chromatography material
and thereby producing the human IgG4 or IgG1 isotype antibody.

One aspect as reported herein is a method for purifying a human IgG4 or IgG1 isotype antibody from a sample comprising the steps of
a) providing a (buffered aqueous) sample comprising a human IgG4 or IgG1 isotype antibody,
b) purifying the human IgG4 or IgG1 isotype antibody with a protein A chromatography method/step, comprising washing the protein A chromatography material with an aqueous solution comprising Histidine, while at least 90% of the bispecific antibody remains bound to the affinity chromatography material.

It has been found that content of a host cell protein can be reduced if an aqueous solution that comprises Histidine is used in a wash step of an affinity chromatography prior to the recovery of the immunoglobulin from the chromatographic material. In one embodiment of all aspects the aqueous solution comprises about 10 mM to about 1000 mM Histidine. In one embodiment of all aspects the aqueous solution comprises about 50 mM to about 400 mM Histidine. In one embodiment of all aspects the aqueous solution comprises about 100 mM to about 300 mM Histidine.

In one embodiment of all aspects the aqueous solution comprises about 200 mM Histidine.

In one embodiment of all aspects the aqueous solution additionally comprises Tris. In one embodiment of all aspects the aqueous solution additionally comprises about 100 mM to about 1500 mM Tris. In one embodiment of all aspects the aqueous solution additionally comprises about 500 mM to about 1300 mM Tris. In one embodiment of all aspects the aqueous solution additionally comprises about 800 mM to about 1200 mM Tris.

In one embodiment of all aspects the aqueous solution comprises Histidine and Tris. In one embodiment of all aspects the aqueous solution comprises 10 mM to about 1000 mM Histidine and about 100 mM to about 1500 mM Tris. In one embodiment of all aspects the aqueous solution comprises 50 mM to about 400 mM Histidine and about 800 mM to about 1200 mM Tris. In one embodiment of all aspects the aqueous solution comprises about 200 mM Histidine and about 1000 mM Tris.

It has been found that the content of a host cell protein can further be reduced if the conductivity of the aqueous solution used in the wash step is low i.e a low conductivity aqueous solution is used for washing. In one preferred embodiment of all aspects the low conductivity aqueous solution has a conductivity value of about 0.5 mS/cm or less. In one embodiment the low conductivity aqueous solution has a conductivity value of from about 0.03 µS/cm to about 0.5 mS/cm. In one embodiment the low conductivity aqueous solution has a conductivity value of from about 0.05 µS/cm to about 0.35 mS/cm. In one embodiment of all aspects the low conductivity aqueous solution is highly purified/deionized water. For some applications deionized water is not suitable to be used in a wash step. In some embodiments the low conductivity aqueous solution is not deionized water.

It has been found that a protein A affinity chromatography can be used for the purposes as reported herein. In one preferred embodiment of all aspects the affinity chromatography is a protein A affinity chromatography. In one embodiment the protein A affinity chromatography is selected from the group comprising MabSelectSure affinity chromatography, ProSep vA affinity chromatography, Mab Capture A affinity chromatography, ProSep Ultra Plus affinity chromatography. In one embodiment the affinity chromatography is a protein G affinity chromatography. In one embodiment the affinity chromatography is an affinity chromatography that uses a recombinant protein as a ligand, that means that the affinity chromatography is a recombinant protein ligand affinity chromatography. In one embodiment the affinity chromatography is an affinity chromatography that uses a single chain Fv as a ligand, that means that the affinity chromatography is a single chain Fv ligand affinity chromatography. In one embodiment the affinity chromatography comprises a mutated Protein A coupled to a chromatography matrix or a fragment of Protein A coupled to a chromatography matrix.

It has been found that the content of (specific) host cell proteins can be reduced. It has been found that especially the content of phospholipase B-like 2 (PLBL2) can be reduced. In one embodiment the (specific) host cell protein is a Chinese hamster ovary (CHO) host cell protein. In one preferred embodiment of all aspects the (specific) host cell protein is phospholipase B-like 2 (PLBL2) or Clusterin. In one embodiment the (specific) host cell protein is phospholipase B-like 2 (PLBL2).

It has been found that low conductivity aqueous solution may comprise Tris or potassium phosphate in low amounts. In one embodiment the low conductivity aqueous solution contains tris(hydroxymethyl)aminomethane (Tris). In one embodiment the low conductivity aqueous solution comprises about 0.1 mM to about 10 mM Tris. In one embodiment the low conductivity aqueous solution comprises about 0.5 mM to about 6.5 mM Tris. In one embodiment the low conductivity aqueous solution comprises about 2 mM Tris. In one embodiment the low conductivity aqueous solution contains potassium phosphate. In one embodiment the low conductivity aqueous solution comprises about 0.05 mM to about 5 mM potassium phosphate. In one embodiment the low conductivity aqueous solution comprises about 0.05 mM to about 2 mM potassium phosphate. In one embodiment the low conductivity aqueous solution comprises about 0.5 mM potassium phosphate.

It has been found that the effect of reducing the content of a host cell protein is pronounced if the low conductivity aqueous solution has a certain pH. In one embodiment the low conductivity aqueous solution has a pH of about 7 or higher. In one embodiment the low conductivity aqueous solution has a pH of about 7.5 or higher. In one embodiment the low conductivity aqueous solution has a pH of from about 7 to about 9.5. In one embodiment the low conductivity aqueous solution has a pH of from about 7.5 to about 8.5. In one embodiment the low conductivity aqueous solution has a pH of about 8. In one embodiment the low conductivity aqueous solution has a pH of about 9.

It has been found that the effect of reducing the content of a host cell protein can also be achieved if the pH of the low conductivity aqueous solution is about 8.5 or higher and the low conductivity aqueous solution has a conductivity value of about 1.2 mS/cm or less. In one embodiment the low conductivity aqueous solution has a pH of about 8.5 or higher and the low conductivity aqueous solution has a conductivity value of about 1.2 mS/cm or less.

In one embodiment the low conductivity aqueous solution is in the pH range of from pH 7 to less than pH 8.5 and has a conductivity value of about 0.5 mS/cm or less and at a pH value of 8.5 or more a conductivity value of about 1.2 mS/cm or less.

It has been found that by the uses and the methods as reported herein the content of host cell proteins like PLBL2 can be reduced to a certain level, e.g. when compared to the load amount of PLBL2 prior to a purification step like an affinity chromatoghraphy step. In one embodiment the content of PLBL2 is reduced at least 20-fold. In one embodiment the content of PLBL2 is reduced at least 40-fold. In one embodiment the content of PLBL2 is reduced at least 50-fold. In one embodiment the content of PLBL2 is reduced at least 90-fold. In one embodiment the content of PLBL2 is reduced at least 100-fold. In one embodiment the content of PLBL2 is reduced at least by 50%. In one embodiment the content of PLBL2 is reduced at least by 66%. In one embodiment the content of PLBL2 is reduced at least by 80%. In one embodiment the content of PLBL2 is reduced at least by 90%. In one embodiment the content of PLBL2 is reduced at least by 95%. In some embodiments the content of PLBL2 is reduced to below 10 ng per mg of antibody. In some embodiments the content of PLBL2 is reduced to below 5 ng per mg of antibody. In some embodiments the content of PLBL2 is reduced to below 2 ng per mg of antibody.

The methods and the uses as reported herein may include one or more further chromatography steps. In one embodiment at least one additional chromatography method/step is performed. In one embodiment an additional ion exchange chromatography method/step is performed. In one embodiment an additional anion exchange chromatography method/step is performed. In one embodiment an additional anion exchange chromatography method/step and an additional cation exchange chromatography method/step are performed.

It has been found that the use of a hydrophobic interaction chromatography step may be omitted. In one embodiment the use or the methods is without an hydrophobic interaction chromatography method/step.

The terms "anti-P-selectin antibody" and "an antibody that binds to P-selectin" or "antibody against P-selectin" refer to an antibody that is capable of binding P-selectin with sufficient affinity such that the antibody is useful as a diagnostic and/or therapeutic agent in targeting P-selectin. In one embodiment, the extent of binding of an anti-P-selectin antibody to an unrelated, non- P-selectin protein is less than about 10% of the binding of the antibody to P-selectin as measured, e.g., by ELISA or surface plasmon resonance. In certain embodiments, an anti- P-selectin antibody binds to an epitope of P-selectin that is conserved among P-selectin from different species. The above also holds for the terms "antibody against factor IXa and factor X" or "antibody against IL-13" or "antibody against amyloid beta".

The specific antibodies to be used in the methods as reported herein are an antibody against P-selectin (anti-P-selectin antibody; inclacumab; IgG4 isotype) as described in WO 2005/100402 or SEQ ID NO: 07 to 12 , a bispecific antibody against factor IXa and factor X (anti-FIXa/X antibody; IgG4 isotype) as described in WO 2012/067176, an antibody against Her2 (anti-Her2 antibody; trastuzumab; IgG1 isotype) as described in WO 1992/022653, a bispecific antibody against angiopoietin 2 (Ang2) and vascular endothelial growth factor A (VEGF-A) (anti-Ang2/VEGF-A antibody; vanucizumab; IgG1 isotype) as described in WO 2011/117329 or SEQ ID NO: 01 to 04, an antibody against amyloid beta (anti-amyloid beta antibody; gantenerumab; IgG1 isotype) as described in WO 2003/070760 or SEQ ID NO: 05 to 06. The terms VEGF or VEGF-A can be used interchangeably herein.

As used herein, the term "binding" or "specifically binding" refers to the binding of the antibody to an epitope of the antigen in an in-vitro assay, preferably in a surface plasmon resonance assay (SPR, BIAcore, GE-Healthcare Uppsala, Sweden). The affinity of the binding is defined by the terms ka (rate constant for the association of the antibody from the antibody/antigen complex), k_{d} (dissociation constant), and K_{D} (k_{d}/kₐ). Binding or specifically binding means a binding affinity (K_{D}) of 10⁻⁷ mol/L or less.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments. A Fab fragement is an antibody fragment obtained by a papain digestion of a (full length/complete) antibody.

Bispecific antibodies" are antibodies which have two different antigen-binding specificities. The term "bispecific" antibody as used herein denotes an antibody that has at least two binding sites each of which bind to different epitopes.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "human IgG isotype antibody" denotes an antibody that comprises a constant region that is derived from a human wild-type IgG isotype, i.e. for example it may comprise a constant region derived from a human IgG isotype with a mutation, e.g. an P329G mutation (numbering according to Kabat).

The term "human IgG4 isotype antibody" denotes an antibody that comprises a constant region that is derived from a human wild-type IgG4 isotype, i.e. for example it may comprise a constant region derived from a human IgG4 isotype with a mutation, e.g. an an P329G mutation and/or S228P, L235E mutation (numbering according to Kabat).

The term "Fc-region" herein is used to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, to the carboxyl-terminus of the heavy chain. However, the C-terminal lysine (Lys447) or the C-terminal glycyl-lysine dipeptide (Gly446Lys447) of the Fc-region may or may not be present. Unless otherwise specified herein, numbering of amino acid residues in the Fc-region or constant region is according to the EU numbering system, also called the EU index, as described in Kabat, E.A. et al., Sequences of Proteins of Immunological Interest, 5th ed., Public Health Service, National Institutes of Health, Bethesda, MD (1991), NIH Publication 91-3242.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. The term "cell" includes cells which are used for the expression of nucleic acids. In one embodiment the host cell is a CHO cell (e.g. CHO K1, CHO DG44), or a BHK cell, or a NS0 cell, or a SP2/0 cell, or a HEK 293 cell, or a HEK 293 EBNA cell, or a PER.C6® cell, or a COS cells. In another embodiment the cell is a CHO cell, or a BHK cell, or a PER.C6® cell. As used herein, the expression "cell" includes the subject cell and its progeny.

The term "washing" denotes the applying of a solution to an affinity chromatography material in order to remove non specifically bound polypeptides and non-polypeptide compounds from the chromatography material, especially to remove host cell protein and host cell DNA. The term "washing" does not encompass the elution of bound material from an affinity chromatography material.

Different methods are well established and widespread used for protein recovery and purification, such as affinity chromatography with microbial proteins (e.g. protein A or protein G affinity chromatography) affinity chromatographie with a recombinant protein as ligand (e.g. single chain Fv as ligand, e.g. Kappa select), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis). These methods can be combined independently in different embodiments as reported herein.

The term "protein A" denotes a protein A polypeptide either obtained from a natural source or produced synthetically.

The term "protein A chromatography material" denotes an inert solid phase to which a protein A is covalently linked.

In one embodiment the protein A chromatography material is selected from MabSelectSure, ProSep vA, Mab Capture A, ProSep Ultra Plus, Mab Select, Mab Select Xtra, Poros A, or ProSep A.

The term "high conductivity aquaeous solution"denotes an aquaeous solution with a high conductivity value. The conductivity value may be about 20 mS/cm or higher. The term "medium conductivity aquaeous solution"denotes an aquaeous solution with a medium conductivity value. The conductivity value may be more than 0.5 mS/cm to less than 20 mS/cm.

The term "low conductivity aquaeous solution"denotes an aquaeous solution with a low conductivity value. The conductivity value may be about 0.5 mS/cm or less. The conductivity value may be about 1.2 mS/cm or less, if the pH is about 8.5 or higher.

The following examples and sequences are provided to aid the understanding of the present invention, the true scope of which is set forth in the appended claims.

**Description of the Sequence Listing**

| | |
|---|---|
| **SEQ ID NO: 01** | variable heavy chain domain VH of <VEGF> |
| **SEQ ID NO: 02** | variable light chain domain VL of <VEGF> |
| **SEQ ID NO: 03** | variable heavy chain domain VH of <ANG-2> |
| **SEQ ID NO: 04** | variable light chain domain VL of < ANG-2> |
| **SEQ ID NO: 05** | variable heavy chain domain VH of anti-amyloid beta antibody (IgG1 isotype) |
| **SEQ ID NO: 06** | variable light chain domain VL of anti-amyloid beta antibody (IgG1 isotype) |
| **SEQ ID NO: 07** | variable heavy chain domain VH1 of anti-P-selectin antibody |
| **SEQ ID NO: 08** | variable heavy chain domain VH2 of anti-P-selectin antibody |
| **SEQ ID NO: 09** | variable heavy chain domain VH3 of anti-P-selectin antibody |
| **SEQ ID NO: 10** | variable light chain domain VL1 of anti-P-selectin antibody |
| **SEQ ID NO: 11** | variable light chain domain VL2 of anti-P-selectin antibody |
| **SEQ ID NO: 12** | variable light chain domain VL3 of anti-P-selectin antibody |

### Example 1

### Material and Methods

### Antibodies

The current invention is exemplified with an antibody against P-selectin (anti-P-selectin antibody; inclacumab; IgG4 isotype) as described in WO 2005/100402 or SEQ ID NO: 07 to 12, with a bispecific antibody against factor IXa and factor X (anti-FIXa/X antibody; IgG4 isotype) as described in WO 2012/067176,with an antibody against Her2 (anti-Her2 antibody; trastuzumab; IgG1 isotype) as described in WO 1992/022653 with a bispecific antibody against Ang2 and VEGF-A (anti-Ang2/VEGF-A antibody; vanucizumab; IgG1 isotype) as described in WO 2011/117329 or SEQ ID NO: 01 to 04, with an antibody against amyloid beta (anti-amyloid beta antibody; gantenerumab; IgG1 isotype) as described in WO 2003/070760 or SEQ ID NO: 05 to 06.

### Detection methods for overall host cell protein (HCP), Phospholipase B-like 2 protein (PLBL2) and Clusterin

### a) CHO HCP assay

The residual CHO HCP content in process samples is determined by an electrochemiluminescence immunoassay (ECLIA) on cobas e 411 immunoassay analyzer (Roche Diagnostics).

The assay is based on a sandwich principle using polyclonal anti-CHO HCP antibody from sheep.

First incubation: Chinese hamster ovary host cell protein (CHO HCP) from 15 µL sample (neat and/or diluted) and a biotin conjugated polyclonal CHO HCP specific antibody form a sandwich complex, which becomes bound to streptavidin-coated microparticles via interaction of biotin with streptavidin.

Second incubation: After addition of polyclonal CHO HCP-specific antibody labeled with ruthenium complex (Tris(2,2'-bipyridyl)ruthenium(II)-complex) a ternary sandwich complex is formed on the microparticles.

The reaction mixture is aspirated into the measuring cell where the microparticles are magnetically captured onto the surface of the electrode. Unbound substances are then removed in a washing step. Application of a voltage to the electrode then induces chemiluminescent emission which is measured by a photomultiplier.

The concentration of CHO HCP in the test sample is finally calculated from a CHO HCP standard curve of known concentration.

### b) CHO PLBL2 assay

The residual Chinese hamster ovary (CHO) Phospholipase B-like 2 protein (PLBL2) content in process samples is determined by an electrochemiluminescence immunoassay (ECLIA) on cobas e 411 immunoassay analyzer (Roche Diagnostics).

The assay is based on a sandwich principle using monoclonal anti-CHO PLBL2 antibody from mouse.

In a first incubation step, CHO PLBL2 from 30 µL sample (neat and/or diluted), biotin labeled monoclonal CHO PLBL2-specific antibody, and a monoclonal CHO PLBL2-specific antibody labeled with a ruthenium complex (Tris(2,2'-bipyridyl)ruthenium(II)-complex) form a sandwich complex.

In a second step after addition of streptavidin-coated microparticles, the ternary complex becomes bound to the solid phase via interaction of biotin and streptavidin.

The reaction mixture is aspirated into the measuring cell where the microparticles are magnetically captured onto the surface of the electrode. Unbound substances are then removed in a washing step. Application of a voltage to the electrode then induces chemiluminescence, which is measured by a photomultiplier.

The concentration of CHO PLBL2 in the test sample is finally calculated from a CHO PLBL2 standard curve of known concentration.

### c) Clusterin assay

The residual Clusterin content in process samples is determined by a commercial assay from which was used according to the manufacturer's instructions.

In brief, this assay is a Sandwich ELISA based, sequentially, on:
1) binding of the rat Clusterin biotinylated capture antibody to the streptavidin coated affinity columns of the Bioaffy 1000nL CD,
2) capture of rat Clusterin molecules from samples to the anti Clusterin antibody,
3) binding of a second dye-labeled anti Clusterin detection antibody to the captured molecules,
4) quantification of the rat Clusterin using the Gyrolab Evaluator.

### Example 2

### Purification of an anti-P-Selectin antibody (IgG4 isotype) in a protein A chromatography

### Antibody: anti-P-Selectin

### General chromatography conditions

Column resin: Protein A material "Mab Select SuRe" (GE-Healthcare) Ø 1cm, Height: 20,1 cm, CV: 15,79 ml
Equipment: Äkta Avant 150
Flow rate: 300 cm/h during all steps
A solution containing an anti-P-Selectin antibody, was applied to a Protein A affinity column after equilibration (step 1) of the column. Initial load of PLBL2 determined in solution containing an anti-P-Selectin antibody: 335 ng PLBL2/mg of antibody. Initial load of Clusterin determined in solution containing an anti-P-Selectin antibody: 2874.8 ng Clusterin/mg of antibody. Initial load of CHOP determined in solution containing an anti-P-Selectin antibody: 100971 ng CHOP/mg of antibody.

The chromatographic steps were performed according to the following general scheme:
Step 1: Equilibration:
Step 2: Load of antibody containing solution
Step 3: Wash I
Step 4: Wash II
Step 5: Wash III
Step 6: Wash IV (additional wash)
Step 7: Elution

After Elution from Protein A affinity column the protein was determined by size exclusion chromatography (SEC) and spectrophotometrically (OD) Analytics.

### SEC:

| | |
|---|---|
| Resin: | TSK 3000 (Tosoh) |
| Column: | 300 x 7,8 mm |
| Flow rate: | 0,5 ml/min |
| Buffer: | 200 mM potassium phosphate containing 250 mM potassium chloride, adjusted to pH 7,0 |
| Wavelength: | 280 nm |

### OD:

| | |
|---|---|
| Specific coefficient: | 1,54 |
| Wavelength: | 280 nm minus 320 nm |

Specific buffer conditions for Protein A chromatography (anti-P-Selectin antibody)
a) Control (wash with equilibration buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | --- |
| *Step 6: Wash IV:* | --- |
| Step 7: Elution: | 50 mM acedic acid, pH 4,0 |

b) low conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

c) low conductivity wash (with potassium phosphate (KP) only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | 25 mM Tris, 25 mM NaCl, pH 7,0 |
| *Step 6: Wash IV:* | 0.5 mM potassium phosphate, pH 8.0 |
| Step 7: Elution: | 50 mM acedic acid, pH 4,0 |

d) high conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

e) low conductivity wash (with Tris buffer only; pH 6.0)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | 25 mM Tris, 25 mM NaCl, pH 7,0 |
| *Step 6: Wash IV:* | 2 mM Tris, pH 6.0 |
| Step 7: Elution: | 50 mM acedic acid, pH 4,0 |

f) high conductivity wash (with Histidine (His)/Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

g) low conductivity Tris + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

h) low conductivity potassium phosphate (KP) + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 0.5 mM potassium phosphate, pH 8.0 |
| Step 7: Elution: | 50 mM acedic acid, pH 4,0 |

i) low conductivity Tris + high conductivity Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

j) low conductivity Tris; pH 6.0 + high conductivity Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | 2 mM Tris, pH 6.0 |
| Step 7: Elution: | 50 mM acedic acid, pH 4,0 |

### Results:

| Run | HCP total [ng/mg] | PLBL2 [ng/mg] | Clusterin [ng/mg] | Yield [%] |
|---|---|---|---|---|
| a | 218 | 105.9 | 27.4 | 93.44 |
| b | 105 | 0.8 | 11.2 | 94.61 |
| c | 114 | 0.7 | 11.8 | 86.67 |
| d | 48 | 14.7 | 23.2 | 89.33 |
| e | 155 | 18.7 | 53.8 | 107.3 |
| f | 106 | 2.9 | 21.6 | 84.9 |
| g | 83 | 0.4 | 11.8 | 85 |
| h | 91 | 0.4 | 9.1 | 80.34 |
| i | 90 | 0.4 | 15.7 | 84.92 |
| j | 141 | 1.5 | 53 | 106.9 |

### Example 3

### Purification of an anti-amyloid beta antibody (IgG1 isotype) in a protein A chromatography

General conditions were according to the conditions described in Example 2.

### Antibody: anti-amyloid beta.

Initial load of PLBL2 determined in solution containing an anti-amyloid beta antibody: 2019.7 ng PLBL2/mg of antibody. Initial load of CHOP determined in solution containing an anti-amyloid beta antibody: 578908 ng CHOP/mg of antibody.

Specific buffer conditions for Protein A chromatography
a) Control (wash with equilibration buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | --- |
| *Step 6: Wash IV:* | --- |
| Step 7: Elution: | 50 mM acedic acid, pH 4,0 |

b) low conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

c) high conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

d) low conductivity Tris + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 6828 | 17.3 | n.d. | 80.3 |
| b | 7794 | 17.8 | n.d. | 73.1 |
| c | 1595 | 1.7 | n.d. | 55.6 |
| d | 6132 | 2.3 | n.d. | 67.3 |

### Example 4

### Purification of an anti-Her2 antibody (IgG1 isotype) in a protein A chromatography

General conditions were according to the conditions described in Example 2.

### Antibody: anti-Her2

Initial load of PLBL2 determined in solution containing an anti-Her2 antibody: 1662.5 ng PLBL2/mg of antibody. Initial load of CHOP determined in solution containing an anti-Her2 antibody: 727070 ng CHOP/mg of antibody.

Specific buffer conditions for Protein A chromatography
a) Control (wash with equilibration buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | --- |
| *Step 6: Wash IV:* | --- |
| Step 7: Elution: | 50 mM acedic acid, pH 4,0 |

b) low conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

c) high conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

d) low conductivity Tris + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 309 | 1.2 | n.d. | 85.5 |
| b | 227 | 1 | n.d. | 77 |
| c | 26 | 0.2 | n.d. | 70.9 |
| d | 42 | 0.5 | n.d. | 83.8 |

### Example 5

### Purification of a bispecific anti-Ang2/VEGF-A antibody (IgG1 isotype) in a protein A chromatography

General conditions were according to the conditions described in Example 2.

### Antibody: anti-Ang2/VEGF-A

Initial load of PLBL2 determined in solution containing a bispecific anti-Ang2/VEGF-A antibody: 919.7 ng PLBL2/mg of antibody. Initial load of CHOP determined in solution containing an anti-Ang2/VEGF-A: 682304 ng CHOP/mg of antibody.

Specific buffer conditions for Protein A chromatography
a) Control (wash with equilibration buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | --- |
| *Step 6: Wash IV:* | --- |
| Step 7: Elution: | 50 mM acedic acid, pH 4,0 |

b) low conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

c) high conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

d) low conductivity Tris + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 3035 | 1.0 | n.d. | 85.0 |
| b | 1707 | 0.8 | n.d. | 79.8 |
| c | 655 | 0.7 | n.d. | 52 |
| d | 1050 | 0.8 | n.d. | 92.3 |

### Example 6

### Purification of a bispecific anti-FIXa/X antibody (IgG4 isotype) in a protein A chromatography

Purification of anti-FIXa/X antibody was tested in two different chromatograhpy settings:

### Setting 1

General conditions were according to the conditions described in Example 2.

### Antibody: anti-FIXa/X

Initial load of PLBL2 determined in solution containing an anti-FIXa/X antibody: 557 ng PLBL2/mg of antibody. Initial load of CHOP determined in solution containing an anti-FIXa/X: 387377 ng CHOP/mg of antibody.

Specific buffer conditions for Protein A chromatography
a) high conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

b) low conductivity Tris + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 1632 | 19.1 | n.d. | 79 |
| b | 2148 | 1.1 | n.d. | 77 |

### Setting 2

### General chromatography conditions

Column resin: Protein A material "Mab Select SuRe" (GE-Healthcare) Ø 1cm, Height: 20,1 cm, CV: 15,79 ml
Equipment: Äkta Avant 150
Flow rate: 300 cm/h during all steps
A solution containing an anti-FIXa/X antibody, was applied to a Protein A affinity column after equilibration (step 1) of the column.

Initial load of PLBL2 determined in solution containing an anti-FIXa/X antibody: 557 ng PLBL2/mg of antibody.

The chromatographic steps were performed according to the following general scheme:
Step 1: Equilibration:
Step 2: Load of antibody containing solution
Step 3: Wash I
Step 4: Wash II
Step 5: Wash III (additional wash)
Step 6: Elution

Specific buffer conditions for Protein A chromatography
a) high conductivity wash (with NaSO4 buffer only)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | --- |
| Step 6: Elution: | 35 mM acedic acid, pH 4,0 |

b) low conductivity wash (Tris 1 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 1 mM Tris, pH 8.0 |
| Step 6: Elution: | 50 mM acedic acid, pH 4,0 |

c) low conductivity wash (Tris 2 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 2 mM Tris, pH 8.0 |
| Step 6: Elution: | 35 mM acedic acid, pH 4,0 |

d) low conductivity wash (Tris 4 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 4 mM Tris, pH 8.0 |
| Step 6: Elution: | 50 mM acedic acid, pH 4,0 |

e) low conductivity wash (Tris 6 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 6 mM Tris, pH 8.0 |
| Step 6: Elution: | 50 mM acedic acid, pH 4,0 |

f) low conductivity wash (Tris 4 mM, pH 7.8) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 4 mM Tris, pH 7.8 |
| Step 6: Elution: | 50 mM acedic acid, pH 4,0 |

g) low conductivity wash (Tris 4 mM, pH 8.2) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 4 mM Tris, pH 8.2 |
| Step 6: Elution: | 50 mM acedic acid, pH 4,0 |

h) low conductivity wash (Tris 2 mM) + high conductivity wash (with Histidine (His)/Tris 1M)

| | |
|---|---|
| Step 1: Equilibration: | 20 mM NaPO4, pH 7,5 |
| Step 2: Load | |
| Step 3: Wash I: | 200 mM His/1000 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 35 mM acedic acid, pH 4,0 |

i) low conductivity wash (Tris 2 mM) + high conductivity wash (Histidine (His)/Tris 0.85 M)

| | |
|---|---|
| Step 1: Equilibration: | 20 mM NaPO4, pH 7,5 |
| Step 2: Load | |
| Step 3: Wash I: | 200 mM His/850 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 50 mM acedic acid, pH 4,0 |

j) low conductivity wash (Tris 2 mM) + high conductivity wash (Histidine (His)/Tris 0.7 M)

| | |
|---|---|
| Step 1: Equilibration: | 20 mM NaPO4, pH 7,5 |
| Step 2: Load | |
| Step 3: Wash I: | 200 mM His/700 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 50 mM acedic acid, pH 4,0 |

k) low conductivity wash (Tris 2 mM) + high conductivity wash (Histidine (His)/Tris 0.55 M)

| | |
|---|---|
| Step 1: Equilibration: | 20 mM NaPO4, pH 7,5 |
| Step 2: Load | |
| Step 3: Wash I: | 200 mM His/550 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 1518 | 204.2 | n.d. | 82 |
| b | 646 | 1 | n.d. | 73.8 |
| c | 737 | 1.2 | n.d. | 79 |
| d | 595 | 1.4 | n.d. | 78.5 |
| e | 685 | 1.8 | n.d. | 79.5 |
| f | 692 | 1.4 | n.d. | 78.2 |
| g | 707 | 1.1 | n.d. | 76.4 |
| h | 299 | 0.5 | n.d. | 79 |
| i | 140 | 0.4 | n.d. | 70 |

| Run | HCP total [ng/mg] | PLBL2 [ng/mg] | Clusterin [ng/mg] | Yield [%] |
|---|---|---|---|---|
| j | 100 | 0.5 | n.d. | 71.9 |
| k | 112 | 0.7 | n.d. | 73 |

## Claims

1. Use of an aqueous solution comprising Histidine and Tris in a wash step of a protein A chromatography for reducing the content of a host cell protein wherein the protein A chromatography is used to purify a human IgG4 or IgG1 isotype antibody, wherein the aqueous solution has a pH of 6.5 or higher.

2. Use according to claim 1, wherein the aqueous solution comprises 10 mM to 1000 mM Histidine.

3. Use according to any one of claims 1 to 2, wherein the aqueous solution comprises 200 mM Histidine.

4. Use according to any one of claims 1 to 3, wherein the aqueous solution comprises 100 mM to 1500 mM Tris.

5. Use according to any one of claims 1 to 4, wherein the aqueous solution comprises 200 mM Histidine and 1000 mM Tris.

6. Use according to any one of claims 1 to 5, wherein the host cell protein is phospholipase B-like 2 (PLBL2) or Clusterin.

7. Use according to any one of claims 1 to 6, wherein the protein A chromatography additionally comprises a wash step with a low conductivity aqueous solution.

8. Use according to claim 7, wherein the low conductivity aqueous solution has a conductivity value of 0.5 mS/cm or less.

9. Use according to any one of claims 7 to 8, wherein the low conductivity aqueous solution comprises 0.1 to 8 mM Tris.

10. Use according to to claim 7, wherein the low conductivity aqueous solution comprises 0.05 to 2 mM potassium phosphate.

11. Use according to any one of claims 7 to 10, wherein the low conductivity aqueous solution has a pH of 7 or higher.

12. Use according to any one of claims 1 to 11, wherein the human IgG4 isotype antibody is an antibody against P-selectin or an antibody against factor IXa and factor X.

13. Use according to any one of claims 1 to 11, wherein the human IgG1 isotype antibody is an antibody against amyloid beta or an antibody against Her2 or an antibody against Ang2 and VEGF-A or an antibody against carcinoembryonic antigen (CEA) and CD3.

14. Method for producing a human IgG4 or IgG1 isotype antibody comprising the following steps
a) cultivating a cell comprising a nucleic acid encoding a human IgG4 or IgG1 isotype antibody,
b) recovering the human IgG4 or IgG1 isotype antibody from the cell or the cultivation medium,
c) contacting the human IgG4 or IgG1 isotype antibody with a protein A chromatography material,
d) washing the protein A chromatography material with an aqueous solution comprising Histidine and Tris that has a pH of 6.5 or higher,
e) recovering the human IgG4 or IgG1 isotype antibody from the protein A chromatography material
and thereby producing the human IgG4 or IgG1 isotype antibody.

15. Method according to claim 14, wherein the aqueous solution comprises 200 mM Histidine.

16. Method according to any one of claims 14 to 15, wherein the aqueous solution comprises 200 mM Histidine and 1M Tris.

17. Method according to any one of claims 14 to 16, wherein the protein A chromatography additionally comprises a wash step with a low conductivity aqueous solution having a conductivity value of 0.5 mS/cm or less.

18. Method according to any one of claims 14 to 17, wherein the host cell protein is phospholipase B-like 2 (PLBL2) or Clusterin.

## Patentansprüche

1. Verwendung einer wässrigen Lösung, umfassend Histidin und Tris, in einem Waschschritt einer Protein-A-Chromatografie zum Reduzieren des Gehalts eines Wirtszellenproteins, wobei die Protein-A-Chromatografie zum Reinigen eines humanen IgG4- oder IgG1-Isotyp-Antikörpers verwendet wird, wobei die wässrige Lösung einen pH von 6,5 oder höher hat.

2. Verwendung nach Anspruch 1, wobei die wässrige Lösung 10 mM bis 1.000 mM Histidin umfasst.

3. Verwendung nach einem der Ansprüche 1 bis 2, wobei die wässrige Lösung 200 mM Histidin umfasst.

4. Verwendung nach einem der Ansprüche 1 bis 3, wobei die wässrige Lösung 100 mM bis 1.500 mM Tris umfasst.

5. Verwendung nach einem der Ansprüche 1 bis 4, wobei die wässrige Lösung 200 mM Histidin und 1.000 mM Tris umfasst.

6. Verwendung nach einem der Ansprüche 1 bis 5, wobei das Wirtszellenprotein Phospholipase B-like 2 (PLBL2) oder Clusterin ist.

7. Verwendung nach einem der Ansprüche 1 bis 6, wobei die Protein-A-Chromatografie zusätzlich einen Waschschritt mit einer wässrigen Lösung mit geringer Leitfähigkeit umfasst.

8. Verwendung nach Anspruch 7, wobei die wässrige Lösung mit geringer Leitfähigkeit einen Leitfähigkeitswert von 0,5 mS/cm oder weniger aufweist.

9. Verwendung nach einem der Ansprüche 7 bis 8, wobei die wässrige Lösung mit geringer Leitfähigkeit 0,1 bis 8 mM Tris umfasst.

10. Verwendung nach Anspruch 7, wobei die wässrige Lösung mit geringer Leitfähigkeit 0,05 bis 2 mM Kaliumphosphat umfasst.

11. Verwendung nach einem der Ansprüche 7 bis 10, wobei die wässrige Lösung mit geringer Leitfähigkeit einen pH von 7 oder höher hat.

12. Verwendung nach einem der Ansprüche 1 bis 11, wobei der humane IgG4-Isotyp-Antikörper ein Antikörper gegen P-Selectin oder ein Antikörper gegen Faktor IXa und Faktor X ist.

13. Verwendung nach einem der Ansprüche 1 bis 11, wobei der humane IgG1-Isotyp-Antikörper ein Antikörper gegen Amyloid beta oder ein Antikörper gegen Her2 oder ein Antikörper gegen Ang2 und VEGF-A oder ein Antikörper gegen karzinoembryonales Antigen (CEA) und CD3 ist.

14. Verfahren zum Produzieren eines humanen IgG4- oder IgG1-Isotyp-Antikörpers, umfassend die folgenden Schritte
a) Kultivieren einer Zelle, umfassend eine Nukleinsäure, die für einen humanen IgG4- oder IgG1-Isotyp-Antikörper kodiert,
b) Gewinnen des humanen IgG4- oder IgG1-Isotyp-Antikörpers aus der Zelle oder dem Kulturmedium,
c) Inkontaktbringen des humanen IgG4- oder IgG1-Isotyp-Antikörpers mit einem Protein-A-Chromatografiematerial,
d) Waschen des Protein-A-Chromatografiematerials mit einer wässrigen Lösung, umfassend Histidin und Tris, die einen pH von 6,5 oder höher hat,
e) Gewinnen des humanen IgG4- oder IgG1-Isotyp-Antikörpers aus dem Protein-A-Chromatografiematerial
und dadurch Produzieren des humanen IgG4- oder IgG1-Isotyp-Antikörpers.

15. Verfahren nach Anspruch 14, wobei die wässrige Lösung 200 mM Histidin umfasst.

16. Verfahren nach einem der Ansprüche 14 bis 15, wobei die wässrige Lösung 200 mM Histidin und 1 M Tris umfasst.

17. Verfahren nach einem der Ansprüche 14 bis 16, wobei die Protein-A-Chromatografie zusätzlich einen Waschschritt mit einer wässrigen Lösung mit geringer Leitfähigkeit mit einem Leitfähigkeitswert von 0,5 mS/cm oder weniger umfasst.

18. Verfahren nach einem der Ansprüche 14 bis 17, wobei das Wirtszellenprotein Phospholipase B-like 2 (PLBL2) oder Clusterin ist.

## Revendications

1. Utilisation d'une solution aqueuse comprenant de l'histidine et du Tris dans une étape de lavage d'une chromatographie de protéine A pour réduire la teneur d'une protéine de cellule hôte dans laquelle la chromatographie de protéine A est utilisée pour purifier un anticorps d'isotype IgG4 ou IgG1 humain, dans laquelle la solution aqueuse a un pH de 6,5 ou plus.

2. Utilisation selon la revendication 1, dans laquelle la solution aqueuse comprend de 10 mM à 1 000 mM d'histidine.

3. Utilisation selon l'une quelconque des revendications 1 à 2, dans laquelle la solution aqueuse comprend 200 mM d'histidine.

4. Utilisation selon l'une quelconque des revendications 1 à 3, dans laquelle la solution aqueuse comprend de 100 mM à 1 500 mM de Tris.

5. Utilisation selon l'une quelconque des revendications 1 à 4, dans laquelle la solution aqueuse comprend 200 mM d'histidine et 1 000 mM de Tris.

6. Utilisation selon l'une quelconque des revendications 1 à 5, dans laquelle la protéine de cellule hôte est la phospholipase B-like 2 (PLBL2) ou la clustérine.

7. Utilisation selon l'une quelconque des revendications 1 à 6, dans laquelle la chromatographie de protéine A comprend de plus une étape de lavage avec une solution aqueuse de faible conductivité.

8. Utilisation selon la revendication 7, dans laquelle la solution aqueuse de faible conductivité a une valeur de conductivité de 0,5 mS/cm ou moins.

9. Utilisation selon l'une quelconque des revendications 7 à 8, dans laquelle la solution aqueuse de faible conductivité comprend de 0,1 à 8 mM de Tris.

10. Utilisation selon la revendication 7, dans laquelle la solution aqueuse de faible conductivité comprend de 0,05 à 2 mM de phosphate de potassium.

11. Utilisation selon l'une quelconque des revendications 7 à 10, dans laquelle la solution aqueuse de faible conductivité a un pH de 7 ou plus.

12. Utilisation selon l'une quelconque des revendications 1 à 11, dans laquelle l'anticorps d'isotype IgG4 humain est un anticorps contre la sélectine P ou un anticorps contre le facteur IXa et le facteur X.

13. Utilisation selon l'une quelconque des revendications 1 à 11, dans laquelle l'anticorps d'isotype IgG1 humain est un anticorps contre l'amyloïde bêta ou un anticorps contre le Her2 ou un anticorps contre l'Ang2 et le VEGF-A ou un anticorps contre l'antigène carcino-embryonnaire (CEA) et le CD3.

14. Procédé de production d'un anticorps d'isotype IgG4 ou IgG1 humain comprenant les étapes suivantes
a) culture d'une cellule comprenant un acide nucléique codant pour un anticorps d'isotype IgG4 ou IgG1 humain,
b) récupération de l'anticorps d'isotype IgG4 ou IgG1 humain de la cellule ou du milieu de culture,
c) mise en contact de l'anticorps d'isotype IgG4 ou IgG1 humain avec un matériel de chromatographie de protéine A,
d) lavage du matériel de chromatographie de protéine A avec une solution aqueuse comprenant de l'histidine et du Tris qui a un pH de 6,5 ou plus,
e) récupération de l'anticorps d'isotype IgG4 ou IgG1 humain du matériel de chromatographie de protéine A
et produisant ainsi l'anticorps d'isotype IgG4 ou IgG1 humain.

15. Procédé selon la revendication 14, dans laquelle la solution aqueuse comprend 200 mM d'histidine.

16. Procédé selon l'une quelconque des revendications 14 à 15, dans lequel la solution aqueuse comprend 200 mM d'histidine et 1 M de Tris.

17. Procédé selon l'une quelconque des revendications 14 à 16, dans lequel la chromatographie de protéine A comprend de plus une étape de lavage avec une solution aqueuse de faible conductivité ayant une valeur de conductivité de 0,5 mS/cm ou plus.

18. Procédé selon l'une quelconque des revendications 14 à 17, dans lequel la protéine de cellule hôte est la phospholipase B-like 2 (PLBL2) ou la clustérine.
